# EUROPÄISCHE PATENTSCHRIFT

(11) **EP 0 763 128 B1**
(45) Veröffentlichungstag und Bekanntmachung des Hinweises auf die Patenterteilung: **01.12.1999**
(21) Anmeldenummer: 95919458.0
(22) Anmeldetag: 16.05.1995
(51) Int. Cl.: C07K 16/46

(54) **VERFAHREN ZUR HERSTELLUNG VON HETEROLOGEN BISPEZIFISCHEN ANTIKÖRPERN**
METHOD FOR PRODUCING HETEROLOGOUS BISPECIFIC ANTIBODIES
PROCEDE DE PREPARATION D'ANTICORPS BISPECIFIQUES HETEROLOGUES

(30) Priorität: 03.06.1994 DE 4419399
(43) Veröffentlichungstag der Anmeldung: 19.03.1997
(73) Patentinhaber: GSF-Forschungszentrum für Umwelt und Gesundheit, GmbH, 85764 Oberschleissheim (DE)
(72) Erfinder: LINDHOFER, Horst, D-82194 Gröbenzell (DE); THIERFELDER, Stephan, D-82229 Eichenau (DE)
(86) Internationale Anmeldenummer: EP9501850
(87) Internationale Veröffentlichungsnummer: WO9533844

(56) Entgegenhaltungen:
- GB-A- 2 197 322
- GB-A- 2 197 323
- INTERNATIONAL JOURNAL OF CANCER, Bd. 55, Nr. 6, 2.Dezember 1993 GENF, DIE SCHWEIZ, Seiten 931-937, S. FERRINI ET AL. 'Targeting of T lymphocytes against EGF-receptor+ tumor cells by bispecific monoclonal antibodies: Requirement of CD3 molecule cross-linking for T-cell activation.'
- COMPTES RENDUS DE L'ACAD MIE DES SCIENCES, Bd. 310, Nr. 9, 26.April 1990 PARIS, Seiten 377-382, V. LEB GUE ET AL. 'Production et caractérisation d,anticorps monoclonaux hybrides présentant une double isotypie IgG1/IgG3.'
- JOURNAL OF THE NATIONAL CANCER INSTITUTE, Bd. 79, Nr. 6, Dezember 1987 BETHESDA, MD, VSA, Seiten 1393-1401, M. CLARK ET AL. 'T-cell killing of target cells induced by hybrid antibodies: Comparison of two bispecific monoclonal antibodies.'
- EXPERIMENTAL HEMATOLOGY, Bd. 22, Nr. 8, 21.August 1994 - 25.August 1994 NEW YORK, NY, VSA, Seite 763 H. LINDHOFER ET AL. 'Rat-mouse quadromas allow augmented generation of bispecific antibodies and single step purification. First in vivo studies.'
- IMMUNOBIOLOGY, Bd. 191, Nr. 2-3, September 1994 STUTTGART, DEUTSCHLAND, Seite 248 H. LINDHOFER ET AL. 'Preferential species-restricted heavy/light chain pairing in rat/mouse quadromas detected during single step purification of bispecific antibodies on protein A.'

## Beschreibung

Die Erfindung betrifft ein Verfahren zur Herstellung von heterologen bispezifischen Antikörpern.

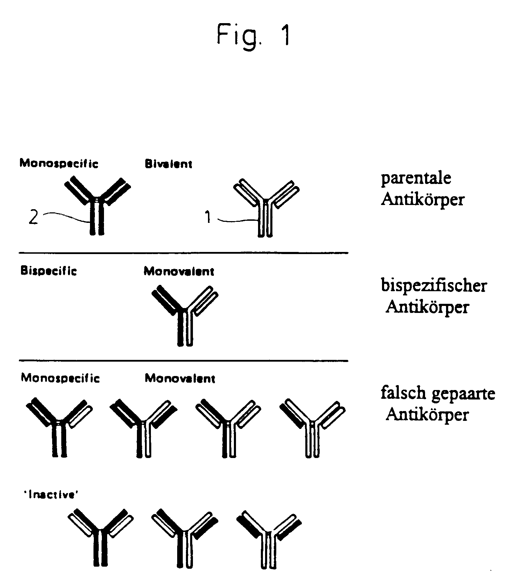

Definitionen einiger unten angeführter Begriffe:
Bispezifische Antikörper bsAk:
   Ein bsAk ist ein Paar, bestehend aus schwerer und leichter Immunglobulinkette, homolog zu einem monoklonalem Antikörper, der gegen ein bestimmtes Antigen gerichtet ist, während das andere schwere/leichte- Immunglobulinkettenpaar homolog zu einem monoklonalen Antikörper ist, der ein anderes Antigen erkennt. Dies resultiert in der Fähigkeit des bsAk zwei unterschiedliche Antigene simultan zu binden.
Heterologe bispezifische Antikörper:
   Als heterolog werden in diesem Zusammenhang bsAk bezeichnet, die schwere/leichte- Immunglobulinkettenpaare unterschiedlicher Species oder Subklasse besitzen.
Quadrom = Hybrid-Hybridom
   Quadrome resultieren aus der Fusion zweier antikörperproduzierender Hybridomzellen.
Parentale Antikörper:
   Parentale Antikörper werden ebenfalls von Quadromen produziert und sind mit den monoklonalen Antikörpern der Ausgangsklone, die zur Herstellung der Quadrome verwendet wurden, identisch (siehe Fig. 1).

Bispezifische Antikörper (bsAk) besitzen eine Vielzahl von Anwendungsbereichen die, von der Immundiagnostik [1] bis zur Therapie (z. B. Immuntherapie von Tumoren, [2]; Unterdrückung von Abstoßungs- und Autoimmunreaktionen, [3]) beim Menschen reichen. Prinzipiell können bsAk durch drei unterschiedliche Verfahren hergestellt werden, mittels chemischer Konjugation [4], mit Hilfe gentechnischer Methoden [5] oder durch die Fusion zweier Hybridomzellinien [6]. BsAk, die durch die letztere Methode hergestellt werden, haben den Vorteil "echte" Antikörper mit einer korrekten Glykosylierung zu sein, die in einer reproduzierbaren Art und Weise von einem klar definiertem Klon gewonnen werden können. Ein wesentlicher Nachteil dieser Methode ist aber, daß die Kulturüberstände derartiger Hybrid-Hybridome bis zu 10 verschiedene Antikörpermoleküle enthalten, die durch die freie Kombination von schweren und leichten Immunglobulinketten entstehen Aus diesem Grund sind relativ aufwendige Reinigungsverfahren zur Isolierung der korrekt gepaarten bispezifischen Antikörper notwendig.

Es gibt eine Reihe von Reinigungsverfahren für bsAk, die fast alle gängigen chromatographischen Methoden nutzen wie z.b Kationen- und Anionen- austauscherchromatographie, hydrophobe Interaktions-Chromatographie, Hydroxylapatit und Affinitätschromatographie. Ein gemeinsames Merkmal all dieser Verfahren ist, daß sich optimale Reinheitsgrade erst nach Einsatz von mindestens zwei unterschiedlichen Trennmechanismen (Säulenpassagen) erreichen lassen. Dies stellt einen wesentlichen Nachteil für eine großtechnische Reinigung dar. Eine Methode, die in letzter Zeit vermehrt eingesetzt wird und diesen Nachteil zu umgehen versucht, soll hier näher diskutiert werden. Dabei handelt es sich um die Kationenaustauscherchromatographie (KA). Diese Trenntechnik beruht auf der unterschiedlichen Ladung von verschiedenen Immunglobulinen bei einem gegebenen saurem pH (meist zwischen pH4,5-5,8) und erlaubt, unter gewissen Voraussetzungen, die Isolierung der bsAk-Fraktion in einem Reinigungsschritt.

Dem Vorteil der Ein-Schritt-Reinigung stehen eine Reihe von Nachteilen gegenüber die vor allem bei einer großtechnischen Reinigung von bsAk ins Gewicht fallen:
a) An eine KA-Säule binden nicht nur Immunglobuline sondern alle sonstigen bei dem gegebenen pH positiv geladenen Proteine (wie z.B bovines Serumalbumin aber auch bovines Immunglobulin), wie aus [8] bekannt;
b) dadurch wird gleichzeitig die Kapazität der Säule für bsAk verringert,
c) dadurch daß die Paarungsmöglichkeit zwischen den schweren und leichten Ketten in Maus/Maus bzw. Ratte/Ratte Quadromen generell nicht eingeschränkt [7] ist gibt es bis zu 10 Antikörpervarianten . KA-Säulen können derartige Varianten aufgrund der Ladungsunterschiede bis zu einem gewißen Grad trennen [8]. Prinzipiell ist dies aber nur bei bsAk möglich, bei denen die parentalen monoklonalen Antikörper sich genügend in den Ladungen ihrer leichten und schweren Ig-Ketten, bei dem gegebenen Puffersystem, das für die Auftrennung verwendet wird, unterscheiden. D. h. für bestimmte bsAk mit ungünstigen Antikörperkombinationen ist eine Aufreinigung mittels KA-Chromatographie nicht möglich.
d) teures Trennmaterial
e) durch vorausgehende Konzentrierungsschritte wie z.B Salzfällungen ist eine teilweise Denaturierung der bsAk möglich.

Des weiteren ist aus Ey et al. [9] bekannt, daß die Subklassen der Maus bei unterschiedlichen pH-Werten von Protein A eluierbar sind. Es wurden auch bereits Versuche unternommen um diese Eigenschaft von Protein A, für die Reinigung der bsAk-Fraktion von Maus/Maus-Quadromen zu verwenden. Die wesentlichsten Nachteile der o. a. Methoden sind folgende:
1. Bei den bisher beschriebenen Reinigungsmethoden [10, 11], werden zunächst alle sezernierten Antikörpervarianten an Protein A gebunden. Daher steht ein Drittel weniger Kapazität zur Bindung der bsAk an die Protein A-Säule zur Verfügung, als beim hier beschriebenen Verfahren.
2. Als Folge dieser Bindung aller, von einem Maus/Maus-Quadrom sezernierten Antikörpervarianten an Protein A, ist die Abtrennung der unerwünschten parentalen Antikörper von der zu reinigenden bsAk-Fraktion, mit Hilfe der sequentiellen pH-Elution, nicht so präzise.
3. Ein wesentlicher Faktor bei der Herstellung und Reinigung von bsAk, ist die Kontrolle der Fehlpaarungen von schweren und leichten Ig-Ketten. Dieser Punkt kann bei Maus/Maus bzw. Ratte/Ratte bsAk-Kombinationen zur unlösbaren Aufgabe werden.

Aus INTERNAT. J. CANCER, Bd. 55, Nr. 6, 1993, S. 931 - 37 ist ein chromatographisches Verfahren zur Reinigung bispezifischer Antikörper bekannt. Nach der dort beschriebenen Methode ist es jedoch nicht möglich die erwünschten Antikörper mittels einer einfachen pH Gradienten Elution zu erhalten, da die jeweiligen Fraktionen nicht entsprechend aufgelöst werden können und die Trennung in einem mühsamen chromatographischen Arbeitsschritt erfolgen muß. Des weiteren sind aus der GB-A-2 197 322, der GB-A-2 197 323 und aus C. R. ACAD. SCI. PARIS, Bd. 310, Nr. 9, 1990, S. 377 - 82 bispezifische Antikörper bekannt, einfache Wege zu deren Herstellung sind jedoch nicht angegeben.

Aufgabe der Erfindung ist es, ein Verfahren der e. g. Art zur Verfügung zu stellen, mit dem es möglich ist heterologe bispezifische Antikörper mit hoher Reinheit herzustellen.

Gelöst wird diese Aufgabe durch die Merkmale des Patentanspruchs 1. Die Unteransprüche beschreiben vorteilhafte Ausgestaltungen des Verfahrens, bzw vorteilhafte Antikörper die mit diesem Verfahren herstellbar sind.

Das Verfahren gemäß dem Patentanspruch 1. hat gegenüber den o. a. Verfahren folgende Vorteile:

Bei den von uns vorgeschlagenen heterologen bsAk-Kombinationen ist der Nachweis, vor allem der beiden leichten Ketten bei denen es ja keine Subklassenunterschiede gibt, was eine Qualitätskontrolle der bsAk entscheidend erleichtert.

Von uns wurde erstmals gefunden, daß in Maus/Ratte Quadromen eine höhere bsAk-Ausbeute durch eine bevorzugte, korrekte schwere/leichte Ig-Kettenpaarung möglich ist.

Da bei unserem Verfahren einer der am bsAk beteiligten parentalen Antikörper, grundsätzlich nicht an Protein A bindet, ist die Diskriminierung der bsAk-Fraktion und der zweiten parentalen Antikörperfraktion die an Protein A bindet, wesentlich besser.Durch diese Entdeckung bekommt die bessere Ausnutzung der Reinigungsmöglichkeit von heterologen bsAk über Protein A, im Vergleich zu Maus/Maus oder Ratte/Ratte bsAk noch eine weitere Dimension. Durch den wesentlich geringeren Anteil an Fehlpaarungen (Verringerung von 70 % auf 10 - 30 % je nach Klonkombinationen, siehe Fig. 1) von schweren und leichten Ig-Ketten, wird ein enormer Betrag an Energie und Kosten (Strom für Brutschränke, Wachstumsmedien usw.) und Kapazität auf der Protein A-Säule, eingespart.

Das Prinzip der Erfindung basiert auf den unterschiedlichen Affinitätskonstanten einer homologen-(CH2-CH3)2 Immunglobulinregion und einer heterologen-(CH2-CH3)(CH2'-CH3') Immunglobulinregion gegenüber Protein A. Dabei trägt bei der hetero-(CH2-CH3) Konfiguration lediglich eine CH2-CH3-Region zur Bindung an Protein A bei. Die zweite CH2'-CH3'-Region kann unter Umständen, nach bereits vollzogener Bindung des bsAk an Protein A diese Bindung noch unterstützen. Die unterschiedlichen Affinitätskonstanten der Immunglobuline mit homo- und hetero-Konfiguration gegenüber Protein A werden zur Reinigung der bispezifischen Komponente genutzt.

Ein wesentlicher Punkt dieses neuen Reinigungsprinzips für bsAk ist, daß einer der parentalen Antikörper, die ebenfalls von den Quadromzellen sezerniert werden, nicht an Protein A bindet. D. h. eine der Hybridomzellinien, die mit einer zweiten Hybridomzellinie zum Quadrom fusioniert wird, sollte einen Antikörper produzieren der nicht an Protein A bindet. Aus dieser Bedingung resultiert auch ein relativ hoher Unterschied zwischen den Affinitätskonstanten des bsAk und dem zweiten parentalen Antikörper (der gut an Protein A binden sollte) gegenüber Protein A, der für eine möglichst saubere Abtrennung dieser beiden Antikörpervarianten von Bedeutung ist. Diese wichtige Voraussetzung wird am effizientesten durch die Fusion einer Mausantikörper produzierenden Zellinie, mit einer Rattenantikörper produzierenden Zellinie erreicht. Von dieser Regel ist die Rattenantikörpersubklasse IgG2c ausgenommen, da sie als einzige der Rattenantikörper eine genügend hohe Affinität zu Protein A besitzt.

Eine weitere Paarungsmöglichkeit, die diese Voraussetzungen erfüllt, ist die Kombination der humanen Subklasse IgG3 (keine Bindung an Protein A) mit einer der humanen Subklassen IgG1, IgG2a und IgG2b.

Ein weiterer Punkt, der von wesentlicher Bedeutung für eine möglichst einfache Reinigung von bsAk ist, ist eine offensichtlich präferentielle Paarung der schweren und leichten Immunglobulinketten innerhalb einer Species in Quadromzellen. D.h. in einem Quadrom, das aus der Fusion eines Ratten-Hybridoms mit einem Maus-Hybridom hervorgegangen ist, paart die leichte Ig-Kette der Maus größtenteils mit der schweren Ig-Kette der Maus und analog die leichte Ig-Kette der Ratte mit der schweren Ig-Kette der Ratte. Die Paarung der schweren Ketten ist dagegen offensichtlich nicht restringiert. Eine Reihe von Untersuchungen, die die freie Kombinationsfähigkeit von schweren und leichten Ketten innerhalb von Maus/Maus-Quadromen untersucht haben, kommen zu dem Ergebnis, daß generell keine Restriktion bezüglich der freien Kombinierbarkeit zwischen den je 2 schweren und leichten Ig-Ketten besteht [7]. D. h. nimmt man die Summe aller von einem Maus/Maus-Quadrom sezernierten Antikörpervarianten als 100 % so schwankt der Prozentsatz der korrekt gepaarten bispezifischen Komponente zwischen 7,5 - 10 % [7, 8].

In den von uns untersuchten heterologen Maus/Ratte Quadromen dagegen steigt der Anteil der korrekt gepaarten bsAk um das 3-fache auf 20 - 30 %. Wobei ein höherer Anteil aufgrund der offensichtlich weiterbestehenden freien Kombination der schweren Ketten untereinander nicht möglich ist.

Zusammengefaßt bringt die Herstellung von heterologen bsAk mit anschließender Reinigung über Protein A folgende Vorteile:
a) ein um das bis zu 3-fache gesteigerter Anteil von korrekt gepaarten bsAk im Vergleich zu konventionellen Maus/Maus oder Ratte/Ratte -Quadromen,
b) falls einer der parentalen Ausgangsantikörper die humane Subklasse IgG3 (oder deren CH2-CH3 Region) besitzt, können selbst bsAk humanisierter Antikörper isoliert werden. Dabei könnte wie in Bsp.2 näher erläutert, die korrekte Paarung der Ratten- und Maus-Fab Domänen, weiterhin genutzt werden,
c) bsAk, die auf parentalen Ausgangsantikörpern mit sehr ähnlichen isoelektrischen Punkten basieren und damit über Kationenaustauscherchromatographie nicht unterscheidbar sind, werden isolierbar,
d) einfache Ein-Schritt Reinigung der bsAk-Komponente über Protein A unter besonders schonenden Bedingungen bei der nur eine sehr geringe bis keine Denaturierung der bsAk auftritt (Elution bei pH 5,6 - 6,0; 100 mM NaCitrat), dadurch besitzen die so gewonnenen bsAk eine neue Qualität,
e) keine Kontamination durch bovine Immunglobuline (im Gegensatz zur KA), da diese erst bei einem pH > 7 unter Hochsalzbedingungen (> 1 M NaCl) an Protein A binden,
f) keine Kontamination durch andere im Serum enthaltenen Proteine (im Gegensatz zur KA), da diese prinzipiell keine Affinität zu Protein A besitzen,
g) Protein A ist im Vergleich zu den anderen chromatographischen Trennmedien mit Abstand das kostengünstigste Trennmedium,
h) Protein A besitzt bereits bei niederen Drücken hervorragende Trenneigenschaften, so daß keine aufwendige HPLC-Technik notwendig ist und die vor allem für großtechnischen Einsatz wesentlich günstigere FPLC-Technik verwendet werden kann.
i) Der Grad der Fehlpaarungen der schweren und leichten Ig-Ketten bei Ratte/Maus Quadromen kann mittels speziesspezifischer Antiseren (die selektiv die leichten und schweren Ketten erkennen), bestimmt werden.

Die Summe all dieser Verbesserungen kompensiert den eingangs erwähnten Nachteil der Quadrom-Technologie der geringen bsAk Ausbeute und und bringt die unbestrittenen Vorteile dieses Verfahrens zum tragen. Das hier vorgestellte Verfahren kann wesentlich dazu beitragen die enormen Mengen von bsAk kostengünstig bereitzustellen, die für einen Durchbruch der bsAk Therapie in der Klinik notwendig sind.

Protein A ist ein Zellwandbestandteil von Staphylococcus aureus und bindet an die Fc Region von Immunglobulinen einer Reihe von Species und Subklassen mit unterschiedlicher Affinität. Es ist eine Polypeptidkette von 42KDa Größe und enthält fünf Domänen. Vier stark homologe Domänen, von denen jede einzelne einer monovalenten Fc-bindenden Domäne entspricht, sind aufeinanderfolgend angeordnet. Mittels Trypsinverdau können aktive Fragmente isoliert werden die diesen homologen Domänen entsprechen und eine Größe von 7 KDa besitzen. Die Fragmente binden an den Fc Teil von Immunglobulinen mit einer Stöchiometrie von 2 : 1. Aus kristallographischen Daten konnte Deisenhofer [9] ableiten, daß ein derartiges Protein A Fragment an einer Stelle zwischen der CH2 und CH3 Region unter Mitwirkung einiger Aminosäurenreste einer C-proximalen Ausstülpung der CH2 Region bindet.

Zusammengefaßt läßt sich sagen, daß offensichtlich zwei Immunglobuline an ein Protein A Molekül binden, wobei vier homologe Domänen des Protein A Moleküls beteiligt sind. Es ist daher möglich als Säulenmaterial Fragmente von Protein A einzusetzen,welches mindestens zwei zusammenhängende Fragmente B (Bindungsdomänen) enthält.

Die Erfindung wird in folgenden an Hand der Figuren mit Hilfe der Beispiele näher erläutert.

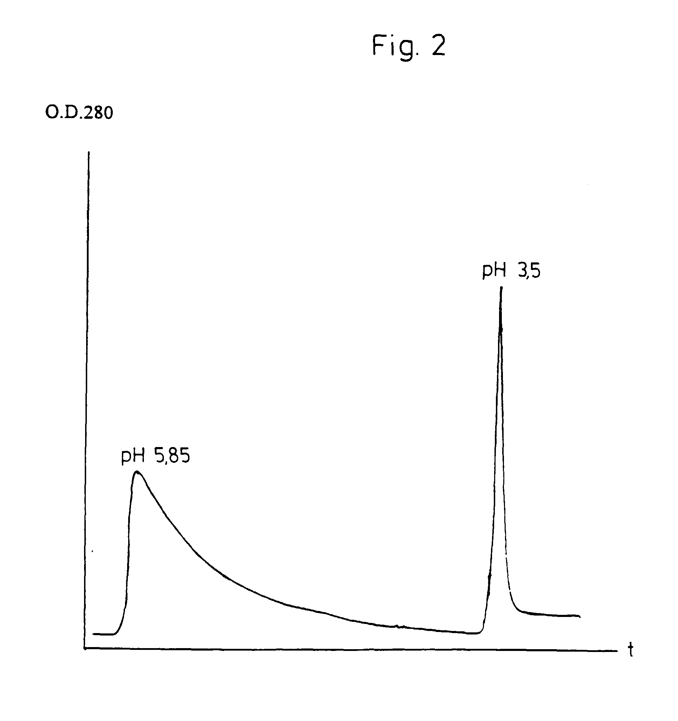

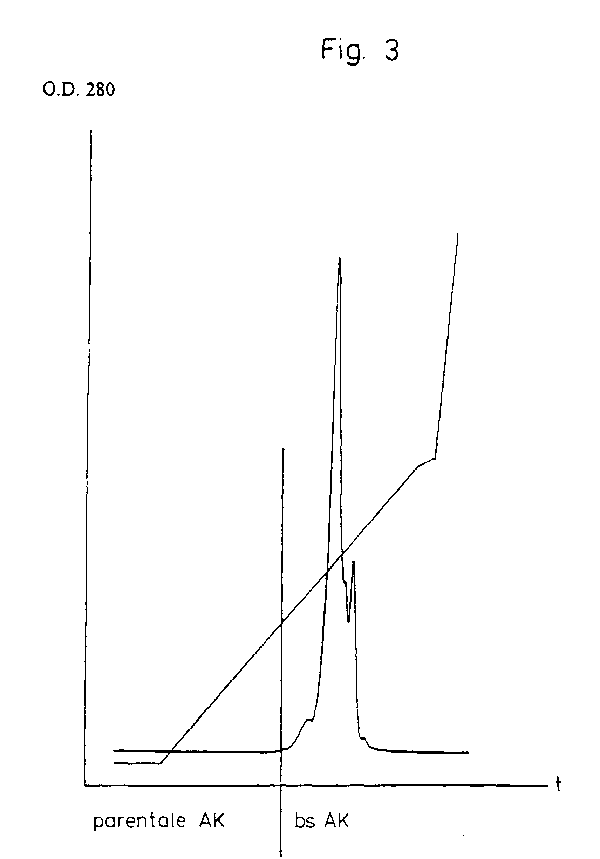

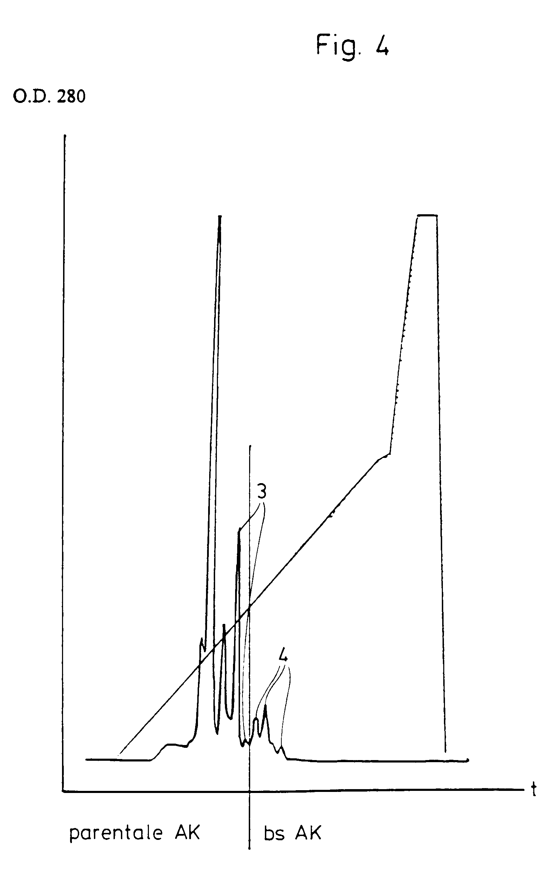

Dabei zeigt die Fig. 1 alle von Hybrid-Hybridomzellen sezernierten Antikörpervarianten. Die Fig. 2 zeigt den Verlauf der bsAk Reinigung über Protein A. Die Fig. 3 und 4 zeigen die Zusammensetzung der beiden Peaks von Fig. 2.

### Beispiel 1

Der erst bsAk wird von einem Quadrom produziert, das durch die Fusion zweier Hybridomzellen unterschiedlicher Spezies hergestellt wurde.

Die Ausgangsklone produzieren 1) einen Anti-Maus-CD3 Rattenantikörper der Subklasse IgG2b sowie 2) einen Anti-Maus-Thy-1.2 Mausantikörper der Subklasse IgG2a.

Ein Liter Kulturüberstand des bsAk wurde auf pH 7 eingestellt und mit einem 0,2 mm Filter sterilfiltriert. Übernacht wurde der so behandelte Kulturüberstand über eine 5ml Protein A - Säule geleitet und anschließend mit 10 Säulenvolumen PBS ungebundene Serumbestandteile sowie die an Protein A nicht bindenden parentalen Rattenantikörper und homo Fc-Varianten entfernt. Mit auf pH 5.9 eingestelltem 0,1M NaCitratpuffer wurde daraufhin der bsAk eluiert. Dabei ist es von Vorteil, wenn die Korngröße des Trägermaterials an welches das Protein A gebunden ist < 50 µm ist. Im Vergleich zu Trägermaterialien mit 100 µm Korngröße konnten auf diese Weise wesentlich schärfere Elutionspeaks erreicht werden. Der relativ hohe pH stellt in diesem Fall sicher, daß sich ausschließlich Antikörpervarianten mit hetero- Fc (CH2-CH3/CH2'-CH3')-Konfiguration im Eluat befinden. Da nur eine Bindungsdomäne des Protein A-Moleküls an die von der Maus stammende CH2-CH3 Region im bsAk bindet, ist bereits ein Absinken des pH von 7 auf 5,85 ausreichend für ein Ablösen der bsAk. Zur abschließenden Regeneration des Protein A's wird auf pH 3,5 justierter 0,1M NaCitratpuffer über die Säule geleitet wodurch sich auch die gebundenen homo- Fc (CH2-CH3)2 Varianten murinen Ursprungs ablösen. Die höhere Bindungsaffinität der parentalen Mausantikörper und der homo-Maus Fc Varianten beruht dabei auf der zusätzlichen zweiten interagierenden Protein A Bindungsdomäne. Der Verlauf der bsAk-Reinigung über Protein A, ist in Fig. 2 gezeigt.

Die Reinheit der bsAk (pH 5,85) - und parentalen Mausantikörper (pH 3,5)- Fraktion wurde mit Hilfe einer Mono S Kationenaustauschersäule (Fig. 3 und 4) sowie im ELISA überprüft.

Durch unterschiedliche Verzuckerungen der Antikörper wird ein Peak welcher einer Protein A Elutionsfraktion entspricht bei der KA-Chromatographie in mehrere Peaks aufgespalten (siehe Fig. 3, bei der der reine bsAk nach Bsp.1, in mehrere Peaks zerfällt).

Fig. 4 zeigt die Zusammensetzung der parentalen Mausantikörper. Fehlpaarungen sind in den Peaks 3 enthalten. BsAk sind in den Peaks 4 als Spurbestandteile enthalten. Aus diesem Diagramm ergibt sich die Erkenntnis daß Fehlpaarungen im Gegensatz zu Maus/Maus-Quadromen wesentlich eingeschränkt vorhanden sind. Daher eignet sich diese modifizierte Quadrommethode bei welcher unterschiedliche Hybridome verschmolzen werden, besonders zur Herstellung von bsAk.

Definition der pH-Bereiche.
1. Unterhalb von pH 5 beginnt die Ablösung der parentalen Maus IgG2a Antikörper. Unterhalb von pH 6.8 beginnt die Ablösung der bsAk.
2. Oberhalb von pH 5.2 keine Ablösung von parentalen IgG2a Mausantikörpern (< 2 %).
   Bei pH 6 quantitative Elution der bsAk, nach längerer Verweildauer des Elutionspuffers mit geringerer bsAk Konzentration.
3. Optimaler Bereich (pH 5.8 ± 0.2) mit kurzer Elutionszeit und maximaler bsAk Konzentration . Es hat sich gezeigt daß Protein A auf grobkörnigem Träger mit Durchmesser > 100 µm schlechter zur Trennung geeignet ist als Protein A auf Träger < als 50 µm.

Anmerkungen zur Kationenaustauscherchromatographie: Die Laufbedingungen der Mono S Läufe waren identisch. Es wurde ein NaCl-Gradient von 50 - 800 mM verwendet, wobei der pH mittels 50 mM MES bei pH 5,5 konstant gehalten wurde.

Abschätzungen der Mengenverhältnisse der einzelnen Antikörpervarianten im Kulturüberstand des heterologen Hybrid-Hybridoms (G2).

Zur Abschätzung der prozentualen Mengenverhältnisse der einzelnen Antikörpervarianten wurden 1. die Flächen unter den einzelnen klar definierten Peaks der Mono S-Läufe herangezogen und 2. die ELISA-Daten der einzelnen Peaks berücksichtigt. Für das Quadrom G2 konnte mit diesen Methoden ein Wert von 20 - 25 % richtig gepaarten bsAk bestimmt werden, was einer Steigerung der bsAk-Ausbeute um das 2,5-fache, gegenüber konventionellen Maus/Maus Quadromen, entspricht. Da ein Wert von 33% für die bsAk-Fraktion aufgrund der freien Paarung von schweren Maus und Ratten Ig-Ketten in der Quadromzelle nicht überschritten werden kann, befindet man sich mit 20 - 25 % Ausbeute bereits nahe der optimalen Ausbeute.

### Beispiel 2a

Dieses Beispiel zeigt, daß selbst innerhalb einer Species die Ein-Schritt Reinigung der bsAk-Komponente über Protein A möglich ist. In diesem Fall muß einer der beteiligten Ausgangsantikörper eine Subklasse besitzen, die nicht an Protein A bindet. In unserem konkreten Beispiel ist der bsAk aus zwei humanisierten Antikörpern zusammengesetzt. Dabei wurden in Klon A die ursprünglich murinen Sequenzen der CH2-CH3 Domänen durch die humanen Sequenzen der Subklasse IgG1 ersetzt. In Klon B dagegen wurden die ursprünglichen Rattensequenzen der CH2-CH3 Domänen durch humane Sequenzen der Subklasse IgG3 ersetzt. Die humane Subklasse IgG3 stellt hierbei den an Protein A nicht-bindenden Anteil des bsAk.
Prinzipiell kann die humane Subklasse IgG1 in diesem Beispiel durch jede weitere an Protein A bindende humane Subklasse ersetzt werden.

Die Reinigung der bsAk aus dem Kulturüberstand eines derartigen Quadroms, kann grundsätzlich nach dem in Bsp.1 gezeigten Protokoll erfolgen.

### Beispiel 2b

In dieser Variante von Beispiel 2a handelt es sich um bsAk zweier vollständig humanisierter Antikörper von denen ein Partner die CH2-CH3 Region der humanen Subklasse IgG3 besitzt. Wenn berücksichtigt wird, daß die andere am bsAk beteiligte humane Subklasse an Protein A bindet, kann das hier vorgestellt Reinigungsprinzip auf einen derartigen humanen Antikörper ebenfalls angewendet werden.

### Beispiel 3

In Beispiel 3 wird gezeigt, daß durch die Fusion geeigneter Ausgangsklone der Vorteil der hier beschriebenen Reinigungsmethode mit einer gewünschten monovalenten Bindung des bsAk kombiniert werden kann. In diesem konkreten Fall würde ein Rattenantikörper mit einer interessanten Spezifität mit einem Mausantikörper, einer in dem jeweiligen in vitro- oder in vivo-System irrelevanten Spezifität, fusioniert werden. Der parentale bivalente Rattenantikörper würde wiederum aufgrund seiner fehlenden Bindung an Protein A bereits bei der Beladung der Protein A-Säule abgetrennt werden. Eine möglichst hohe Ausbeute ist in diesem Beispiel ebenfalls aufgrund der präferentiellen Paarung der schweren und leichten Kette der Ratte miteinander gewährleistet. Ein weiterer Vorteil der hier beschriebenen Variante ist die durch die Maussubklasse IgG2a hinzugewonnene Effektorfunktion des bsAk, falls der beteiligte Rattenantikörper der Subklasse IgG2a oder IgG1 angehören sollte.

### Beispiel 4

In Beispiel 4 wird die Kombination eines, gegen ein humanes Antigen gerichteten monoklonalen Antikörpers, mit einem gegen ein murines Antigen gerichteten Antikörpers, behandelt .Ein derartiger bsAk könnte
1. durch geeignete Wahl der Species bzw. Subklassen der beteiligten monoklonalen Antikörper, die Vorzüge der hier vorgestellten Erfindung zur Reinigung von bsAk sowie der erhöhten, korrekten schwere/leichte Immunglobulinkettenpaarung bei Maus/Ratte-Quadromen, nutzen,
2. aufgrund seiner eingangs erwähnten Spezifitäten im Menschen bzw. der Maus jeweils nur mit einem Bindungsarm (monovalent) binden. Eine Eigenschaft die für bestimmte Therapieformen, wie z. B. die Depletion von bestimmtem Lymphozytenpopulationen, von Bedeutung ist,
3. durch geeignete Subklassenwahl der beteiligten Antikörper bestimmte Effektorfunktionen besitzen,
4. präklinisch auf seine Biokompatibilität in der Maus getestet werden und damit Rückschlüsse auf seine Eigenschaften im Menschen gezogen werden.

### Beispiel 5

Nachdem Rousseaux 1981 [13] gezeigt hat, daß die Rattensubklasse IgG2c als einzige der Rattensubklassen an Protein A bindet, könnten bsAk von Ratte/Ratte Quadromen, bei denen einer der Fusionspartner die Rattensubklasse IgG2c produziert, ebenfalls nach dem in Bsp 1 vorgestellten Protokoll gereinigt werden. Dies wäre gleichzeitig die erste Reinigungsmethode für bsAk der Ratte, mittels Protein A.

### Literatur

[1]
   Karawajew L., Micheel B., Behrsing O., Gaestel M. (1987) J.Immunol. Methods 96:265
[2]
   Nitta T., Sato K., Yagita H., Okumura K., Ishii S. (1990) Lancet 335:368
[3]
   MacLean J. A., Su Z., Guo Y., Sy M., Colvin R. B., Wong J. T. (1993) J.Immunol. 150:1619-1628
[4]
   Brennan M., Davison P. F., Paulus H. (1985) Preparation of bispecific antibodies by chemical recombination of monoclonal immunglobulin G1-fragments. Science 229:81
[5]
   Kostelny S. A., Cole M. S., Tso J. Y. (1992) J. Immunol. 148:1547-1553
[6]
   Clark M., Gilliland L., Waldmann H. (1988) Hybrid antibodies for therapy. Monoclonal antibody therapy. Prog Allergy. Basel, Karger, vo145:31-49
[7]
   De Lau W. B. M., Heije K., Neefjes J. J., Oosterwegel M., Rozemuller E., Bast B. J. E. G. (1991) Absence of preferential homologous H/L chain association in hybrid-hybridomas. J. Immunol.146:906-914
[8]
   Link B. K., Weiner G. J. (1993) Production and characterization of bispecific IgG capable of inducing T-cell mediated lysis of malignant B cells. Blood 81:3343-3349
[9]
   Ey P. L., Prowse S. J., Jenkin C. R. (1978) Isolation of pure IgG1, IgG2a and IgG2b immunoglobulins from mouse serum using protein A-Sepharose. Immunochemistry 15:429-436
[10]
   Couderc J., Kazatchkine M. D., Ventura M., Duc H. T., Maillet F., Thobie N., Liacopoulos P. (1985) Activation of the human classical complement pathway by a mouse monoclonal hybrid IgG1-2a monovalent anti-TNP antibody bound to TNP-conjugated cells. J. Immunol. 134:486-491
[11]
   Kuppen P. J. K. et al. (1993) The development and purification of a bispecific antibody for lymphokine-activated killer cell targeting against the rat colon carcinoma CC531. Cancer Immunol. Immunother. 36:403-408
[12]
   Deisenhofer J. (1981) Crystallographic refinement and atomic models of a human Fc fragment and its complex with fragment B of protein A from Staphylococcus aureus at 2.9 and 2.8 A resolution. Biochemistry 20:2361-2370
[13]
   Rousseaux J., Rousseaux-Prevost R., Bazin H., Biserte G. (1981) Tryptic cleavage of rat IgG: a comperative study between subclasses. Immunol. Lett. 3:93

## Patentansprüche

1. Verfahren zur Herstellung von heterologen bispezifischen Antikörpern mit folgenden Verfahrensschritten:
a) Erzeugung eines Quadroms fusioniert aus Hybridomen von denen eines Antikörper (1) produziert, welche eine Affinität zur Bindungsdomäne von Protein A besitzen, wobei der Antikörper (1) ein Mausantikörper, ein humaner oder humanisierter Antikörper der Subklasse IgG1, IgG2, IgG4 oder ein Rattenantikörper der Subklasse IgG2c ist und von denen das andere Hybriddom Antikörper (2) produziert, welche eine im Vergleich zum Antikörper (1) eine schwächere oder keine Affinität zur Bindungsdomäne von Protein A besitzen, wobei der Antikörper (2) ein Rattenantikörper der Subklasse IgG1, IgG2a, IgG2b, IgG3 oder ein humaner oder humanisierter Antikörper der Subklasse IgG3 ist,
b) Vermehrung und Kultivierung der Quadrome in üblicher Weise,
c) Auftragen des Quadrom-Kulturüberstands auf eine Säule, welche mit einem Material beschichtet ist, das Bindungsdomänen von Protein A als funktionelle Gruppen enthält,
d) Auswaschen der nicht gebundenen Proteine im entsprechenden pH-Bereich und
e) Elution der bsAk in einem pH-Bereich der mindestens 0,5 Einheiten über dem pH liegt, bei dem die Antikörper mit stärkeren Affinität zur Bindungsdomäne von Protein A noch gebunden sind.

2. Verfahren nach Patentanspruch 1, dadurch gekennzeichnet, daß der Mausantikörper ein Antikörper der Subklasse IgG2a, IgG2b oder IgG3 ist.

## Claims

1. Method of producing heterologous bispecific antibodies, having the following method steps:
a producing a quadroma fused from hybridomas, one of which produces antibodies (1) which have an affinity to the binding site of protein A, the antibody (1) being a mouse antibody, a human or humanised antibody of the sub-class IgG1, IgG2, IgG4 or a rat antibody of the sub-class IgG2c, and the other hybridoma of which hybridomas produces antibodies (2) which have a weaker affinity, compared with antibody (1), or no affinity to the binding site of protein A, the antibody (2) being a rat antibody of the sub-class IgGl, IgG2a, IgG2b, IgG3 or a human or humanised antibody of the sub-class IgG3;
b) increasing and cultivating the quadromas in conventional manner;
c) applying the excess quadroma culture to a column coated with a material which contains binding sites of protein A as functional groups;
d) growing the non-bonded proteins in the appropriate pH range; and
e) eluting the bsAk in a pH range, which lies at least 0.5 units above the pH at which the antibodies with a stronger affinity to the binding site of protein A are still bonded.

2. Method according to claim 1, characterised in that the mouse antibody is an antibody of the sub-class IgG2a, IgG2b or IgG3.

## Revendications

1. Procédé de production d'anticorps bispécifiques hétérologues avec les étapes de procédé suivantes :
a) obtention d'un Quadrome fusionné à partir d'hybridomes par lesquels un anticorps (1) est produit, qui possèdent une affinité pour les sites de liaison de la protéine A pour lesquels l'anticorps (1) est un anticorps de souris, un anticorps humain ou humanisé de la sous-classe IgG1, IgG2, IgG4, ou un anticorps de rat des sous-classes IgG2 et par lesquels l'autre hybridome produit des anticorps (2) qui possèdent une affinité en comparaison avec les anticorps (1) plus faible ou pas d'affinité pour les sites de liaison de la protéine A dans lequel l'anticorps (2) est de l'anticorps de rat de la sous-classe IgG1, IgG2a, IgG2b, IgG3 ou un anticorps humain ou humanisé de la sous-classe IgG3,
b) propagation et mise en culture des quadromes d'une manière usuelle,
c) application du résidu de culture du Quadrome sur une colonne qui est enduite avec un matériau qui renferme les sites de liaison de la protéine A en tant que groupes fonctionnels,
d) lavage complet des protéines non liées dans la zone de pH correspondante et,
e) élution des bsAk dans une zone de pH qui se situe au moins 0,5 unité au-dessus du pH auquel les anticorps qui ont une affinité plus forte pour les sites de liaison de la protéine A, sont encore liés.

2. Procédé selon la revendication 1,
caractérisé en ce que
l'anticorps de souris est un anticorps de la sous-classe IgG2a, IgG2b ou IgG3.
